Europäisches Patentamt

European Patent Office

Office européen des brevets

(11) Veröffentlichungsnummer: **0 405 289 A1**

(12) **EUROPÄISCHE PATENTANMELDUNG**

(21) Anmeldenummer: 90111508.9

(22) Anmeldetag: 19.06.90

(51) Int. Cl.⁵: **C07C 309/04, C07C 303/02**

(30) Priorität: 28.06.89 DE 3921130

(43) Veröffentlichungstag der Anmeldung:
02.01.91 Patentblatt 91/01

(84) Benannte Vertragsstaaten:
GR

(71) Anmelder: Henkel Kommanditgesellschaft auf
Aktien
Henkelstrasse 67
D-4000 Düsseldorf 13(DE)

(72) Erfinder: Wüst, Willi, Dr.
Fasanenring 32
D-4030 Ratingen 6(DE)
Erfinder: Lohr, Christoph, Dr.
Liboristrasse 45
D-4600 Dortmund 1(DE)
Erfinder: Eskuchen, Rainer, Dr.
Benrather Schlossallee 36
D-4000 Düsseldorf 13(DE)
Erfinder: Leischner, Hasso
Böcklinstrasse 10
D-4000 Düsseldorf(DE)

(54) Verfahren zur Herstellung von niederen Alkansulfonsäuren aus ihren Alkalisalzen.

(57) Beschrieben wird ein Verfahren zur Herstellung von niederen Alkansulfonsäuren aus ihren Alkalisalzen durch deren Umsetzung mit HCl und Auftrennung des Reaktionsgemisches. Das neue Verfahren ist dadurch gekennzeichnet, daß man zur Gewinnung einer an Chloridionen praktisch freien Alkansulfonsäure deren Alkalisalze, gewünschtenfalls in Abmischung mit Alkalichlorid, in Form einer aufkonzentrierten wäßrigen Salzaufschlämmung mit einem mehrfach-molaren Überschuß an HCl - bezogen auf Alkansulfonsäure - versetzt, die jetzt vorliegende Feststoffphase abtrennt, aus der isolierten Flüssigphase den HCl-Überschuß gewünschtenfalls zusammen mit einem Anteil des Wassers destillativ abzieht und die praktisch chloridfreie Alkansulfonsäure zusammen mit dem Restwasser als Sumpfphase der Destillation gewinnt:

# VERFAHREN ZUR HERSTELLUNG VON NIEDEREN ALKANSULFONSÄUREN AUS IHREN ALKALISALZEN

Die Erfindung betrifft ein verbessertes Verfahren zur Herstellung niederer Alkansulfonsäuren hoher Reinheit aus ihren Alkalisalzen. Erfindungsgemäß lassen sich insbesondere im wesentlichen chloridfreie Säuren der genannten Art herstellen, obwohl der preisgünstige Chlorwasserstoff zur Freisetzung der Alkansulfonsäure aus ihrem Alkalisalz zum Einsatz kommt.

Kurzkettige 1-Sulfonsäuren, insbesondere C1- bis C4-Sulfonsäuren sind für die technische Reinigung als Substitut für Phosphorsäure und/oder Salpetersäure in insbesondere phosphatfreien Reinigungsmitteln vorgeschlagen worden. Zur Zeit stehen für den Einsatzzweck geeignete Sulfonsäurequalitäten zu realistischen Preisen in großtechnischen Mengen jedoch nicht zur Verfügung. Für die Verwendung in technischen Reinigungsmitteln kommen nur Säuren in Betracht, die praktisch frei von Chloridionen sind. Deren Gewinnung ist nach bisherigen Verfahren nur unter einem so beträchtlichen Aufwand möglich, daß an sich besonders interessante Vertreter der niederen Alkansulfonsäuren, beispielsweise die Methansulfonsäure lediglich als teuere Spezialchemikalie käuflich sind.

Zur Herstellung niederer Alkansulfonsäuren sind verschiedene Verfahren vorgeschlagen worden. So können kurzkettige Säuren dieser Art durch Oxidation der entsprechenden Mercaptane mit Salpetersäure oder Persäuren sowie Jod in Gegenwart von Bromidionen in Dimethylsulfoxid hergestellt werden. Diese Verfahren ergeben jedoch nur mäßige Ausbeuten. Zudem sind Mercaptane für großtechnische Verfahren zu teuer.

Kurzkettige Alkansulfonsäuren lassen sich weiterhin aus Alkanen durch Sulfochlorierung mit anschließender Verseifung des Alkansulfonylchlorids oder durch Sulfoxidation herstellen. Die Sulfonierung erfolgt jedoch an der Kohlenwasserstoffkette in statistischer Verteilung und zudem gegebenenfalls auch mehrfach, so daß die selektive Herstellung von 1-Sulfonsäuren nicht möglich ist.

Auch die Umsetzung von Olefinen mit Natriumhydrogensulfit und anschließender Freisetzung der Alkansulfonsäuren gibt unerwünschte Nebenprodukte.

Aus Bl. Chem. Soc. Japan 32, 850 (1959) ist die Umsetzung von kurzkettigen Alkylhalogeniden mit Natriumsulfit zu den entsprechenden Alkylnatriumsulfonaten nach der Strecker-Synthese bekannt. Die Freisetzung der kurzkettigen Alkansulfonsäuren aus den erhaltenen Natriumsalzen ist jedoch nicht beschrieben. Sie stößt auch auf Schwierigkeiten, weil sowohl die kurzkettigen Alkansulfonsäuren als auch die Salze derselben in Wasser gut löslich sind. Der Versuch einer Flüssig/Flüssig-Extraktion führt zu keinem brauchbaren Ergebnis.

Gegenstand der älteren Anmeldung P 38 12 846.2 (D 7989) "Verfahren zur Herstellung von $C_1$ bis $C_6$-Alkansulfonsäuren" der Anmelderin ist ein Verfahren zur Herstellung geradkettiger oder verzweigter $C_1$ bis $C_5$-Alkansulfonsäuren aus ihren Alkalisalzen, das dadurch gekennzeichnet ist, daß man die Alkalisalze in einer Lösung oder Suspension in $C_{1-4}$-Monoalkanolen mit Chlorwasserstoff umsetzt, die ausgefallenen Alkalichloride abtrennt und die $C_{1-6}$-Alkansulfonsäuren aus der Monoalkanolphase isoliert. Wenn auf diese Weise auch freie Säuren der gewünschten Art aus ihren Salzen gewonnen werden können, so ist gleichwohl eine substantielle Verfahrensvereinfachung nach wie vor erwünscht.

Die vorliegende Erfindung geht von der überraschenden Feststellung aus, daß auf das Arbeiten in alkoholischen Flüssigphasen im Sinne der Lehre der zuletzt genannten älteren Anmeldung bei der Freisetzung der Alkansulfonsäure aus ihren Alkalisalzen verzichtet und damit die alkoholische Flüssigphase durch eine wäßrige Reaktionsphase ersetzt werden kann, wenn man unter den im nachfolgenden geschilderten, bestimmt ausgewählten Verfahrensbedingungen arbeitet.

Gegenstand der Erfindung ist dementsprechend ein Verfahren zur Herstellung von niederen Alkansulfonsäuren aus ihren Alkalisalzen durch deren Umsetzung mit Chlorwasserstoff und Auftrennung des Reaktionsgemisches, wobei das neue Verfahren dadurch gekennzeichnet ist, daß man zur Gewinnung einer an Chloridionen praktisch freien Alkansulfonsäure deren Alkalisalze, gewünschtenfalls in Abmischung mit Alkalichlorid, in Form einer aufkonzentrierten wäßrigen Salzaufschlämmung mit einem mehrfach-molaren Überschuß an Chlorwasserstoff - bezogen auf Alkansulfonsäure - versetzt, die jetzt vorliegende Feststoffphase abtrennt, aus der isolierten Flüssigphase den Chlorwasserstoff-Überschuß gewünschtenfalls zusammen mit einem Anteil des Wassers destillativ abzieht und die praktisch chloridfreie Alkansulfonsäure zusammen mit dem Restwasser als Sumpfphase der Destillation gewinnt.

Kern des erfindungsgemäßen Handelns ist die überraschende Feststellung, daß durch Einsatz eines beträchtlichen Überschusses an HCl bei der Umwandlung des in wäßriger Lösung bzw. Aufschlämmung vorliegenden Alkalisalzes der Alkansulfonsäuren unter Freisetzung der freien Säure und gleichzeitiger Bildung des Alkalichlorids die Löslichkeit des Alkalichlorids in der aufkonzentrierten wäßrigen Phase so stark abgesenkt werden kann, daß die praktisch quantitative Entfernung des Alkalichlorids durch einfache Phasentrennung gelingt. In der auf diese Weise zu gewinnenden wäßrigen Flüssigphase liegt die freie Alkansulfonsäure zusammen mit dem eingesetzten HCl-Überschuß vor. Auf die im nachfolgenden noch zu schildernde Weise gelingt unter schonenden Bedingungen die Abtrennung des Chlorwasserstoffs, dessen Restmengen als wäßriges Azeotrop auf destillativem Wege abgezogen werden können, so daß schließlich eine hellfarbige und einstellbare Restwassermengen enthaltende freie Alkansulfonsäure gewonnen werden kann, die praktisch chloridionenfrei ist.

Das Verfahren eignet sich insbesondere für die Herstellung von C1- bis C6-Alkansulfonsäuren und hat besondere Bedeutung für Verbindungen dieser Art mit 1 bis 4 C-Atomen. Insbesondere die Methansulfonsäure läßt sich aus ihren Alkalisalzen durch das neue Verfahren in einfacher Weise gewinnen. Die bevorzugten Alkalisalze sind im Rahmen der erfindungsgemäß geschilderten Umsetzung die Natriumsalze.

Die entscheidende Arbeitshilfe, durch die das Arbeiten in wäßrigem Medium beim hier geschilderten Reaktionstyp möglich wird, ist der Einsatz des Chlorwasserstoffs im Schritt der Freisetzung der Alkansulfonsäure in mehrfach molarem Überschuß. Vorzugsweise wird Chlorwasserstoff wenigstens in etwa 3-molarer Menge bezogen auf vorliegendes Alkalisalz der Alkansulfonsäure verwendet. Dieser HCl-Überschuß ist für die Gesamtbilanz des Verfahrens unproblematisch, weil die zur Salzbildung nicht benötigten Anteile des Chlorwasserstoffs im Kreislauf wieder in die nächste Verfahrensstufe zurückgeführt werden können. Üblicherweise wird mit HCl-Mengen von höchstens etwa 5 Mol - bezogen auf das Alkalisalz der Alkansulfonsäure - gearbeitet. Als besonders geeignet hat sich ein Bereich von etwa 3,2 bis 4 Mol HCl erwiesen.

Nach der Behandlung des als wäßrige Salzaufschlämmung eingesetzten Alkalisalzes der Alkansulfonsäure mit dem Chlorwasserstoff, der in freier Form und/oder als wäßrige Lösung - beispielsweise als rauchende Salzsäure - eingesetzt werden kann, bildet sich eine Aufschlämmung von Alkalichlorid insbesondere Natriumchlorid in einer Flüssigphase aus, die die freie Alkansulfonsäure zusammen mit HCl gelöst enthält. Die Löslichkeit des Natriumchlorids in dieser Flüssigkeit ist durch HCl-Überschuß verschwindend gering. Das als Feststoff vorliegende Natriumchlorid wird in geeigneter Weise beispielsweise durch Filtration oder Zentrifugieren von der Flüssigphase abgetrennt. Diese Trennung kann bei Normaltemperatur oder bestenfalls mäßig erhöhten Temperaturen, also beispielsweise im Temperaturbereich bis etwa 60 °C durchgeführt werden. Häufig liegen zum Zeitpunkt der Phasentrennung Aufschlämmungen im Temperaturbereich von etwa 40 bis 60 °C vor, die sich bei diesen Temperaturen für die Trennstufe eignen.

Die nachfolgende Auftrennung der gewonnen Flüssigphase kann auf destillativem Wege, und zwar insbesondere in mehrstufiger Form erfolgen. In der einfachsten Form wird hier eine zweistufige destillative Trennung derart vorgenommen, daß zunächst der Chlorwasserstoff abgetrieben und in das Hauptverfahren zurückgeführt wird. In der Endstufe dieser Auftrennung der Flüssigphase kann dann ein HCl/Wasser-Gemisch abgetrennt werden. Bevorzugt wird hier im schwachen Vakuum gearbeitet, so daß Temperaturen unter 100 °C eingehalten werden können, wobei insbesondere Temperaturen der Flüssigphase im Bereich von etwa 80 bis 90 °C geeignet sein können. Auch dieses in zweiter Verfahrensstufe abgetrennte HCl/Wasser-Azeotrop kann in die Hauptreaktion zurückgeführt werden, es ist dann jeweils nur der im Kreislauf geführte Wasserbetrag mit zu berücksichtigen.

Nach Abtrennung des Chlorwasserstoffs und begrenzter Wasser mengen aus der Flüssigphase fällt als Sumpfphase die freie Alkansulfonsäure als hellfarbiges chloridfreies Reaktionsprodukt an. In einer bevorzugten Ausführungsform der Erfindung wird dabei die Wasserbilanz des Gesamtverfahrens so geregelt, daß die hier anfallende freie Alkansulfonsäure auch ihrerseits noch einen geringen Restwassergehalt von wenigstens etwa 1 bis maximal etwa 30 Gew.-% und bevorzugt von etwa 10 bis 20 Gew.-% aufweist. Hierzu ist es lediglich notwendig, den Wassergehalt der für die Freisetzungsstufe der Alkansulfonsäure eingesetzten wäßrigen Salzaufschlämmung so zu wählen, daß trotz Abziehens eines Anteils der wäßrigen Phase mit den restlichen überschüssigen HCl-Mengen bei der vollständigen Entfernung der HCl auf destillativem Wege der angestrebte Wassergehalt in Mischung mit der freigesetzten Alkansulfonsäure zurückbleibt.

Die so gewonnenen hochkonzentrierten Alkansulfonsäuren können - gewünschtenfalls nach Verdünnung mit weiterem Wasser - dem jeweiligen Verwendungszweck zugeführt werden. Der Chloridgehalt der Alkansulfonsäuren liegt im akzeptablen Spurenbereich. So ist es beispielsweise ohne weiteres möglich, auf dem geschilderten Weg Methansulfonsäure aus ihrem Natriumsalz mit Chloridgehalten deutlich unter 500 ppm zu gewinnen.

Die zuvor geschilderte destillative Abtrennung der überschüssigen Salzsäure, insbesondere in der Form eines wäßrigen Azeotrops kann beispielsweise in Fallfilmverdampfern erfolgen, die materialmäßig so ausgelegt sind, daß sie durch das an sich aggressive Medium nicht geschädigt werden. Materialien bzw. Vorrichtungen dieser Art sind im Stand der Technik bekannt. Zu nennen sind hier beispielsweise entsprechende Vorrichtungen auf Graphitbasis.

**Beispiele**

Beispiel 1

Eingesetzt wurden 300 g eines Salzgemisches bestehend aus 145 g Natriummethansulfonat, 144 g NaCl und Wasser, welches aus der Reaktion von Methylchlorid mit wäßriger Sulfitlösung und nachfolgender Einengung am Rotationsverdampfer stammte. Das Salzgemisch wurde in 450 g rauchender Salzsäure (37 %) gelöst und das ausgefallene NaCl abfiltriert. Die nachfolgende Einengung des Filtrats (bestehend aus Salzsäure und Methansulfonsäure) am Rotationsverdampfer (T = 110 °C, P = 20 Torr) auf eine Konzentration von 80 Gew.-% Methansulfonsäure und 20 % Wasser ergab eine Lösung mit einem Chloridgehalt von weniger als 200 ppm Cl⁻. Die Ausbeute an Methansulfonsäure betrug 98 % bezogen auf das eingesetzte Methansulfonat.

Beispiel 2

Es wurden 500 g eines analog zu Beispiel 1 hergestellten Salzgemisches bestehend aus 145 g Methansulfonat, 144 g NaCl und Wasser eingesetzt. Über eine Glasfritte wurden in diese Lösung 150 g gasförmige HCl eingeleitet und das ausgefallene NaCl abfiltriert. Die nachfolgende Aufkonzentrierung des Filtrats (Methansulfonsäure und Salzsäure) am Rotationsverdampfer (T = 110 °C, P = 20 Torr) auf eine Konzentration von 80 Gew.-% Methansulfonsäure und 20 Gew.-% Wasser ergab eine Lösung mit einem Chloridgehalt von weniger als 200 ppm Cl⁻. Die Ausbeute betrug 98 % Methansulfonsäure bezogen auf eingesetztes Methansulfonat.

**Ansprüche**

1. Verfahren zur Herstellung von niederen Alkansulfonsäuren aus ihren Alkalisalzen durch deren Umsetzung mit HCl und Auftrennung des Reaktionsgemisches, dadurch gekennzeichnet, daß man zur Gewinnung einer an Chloridionen praktisch freien Alkansulfonsäure deren Alkalisalze, gewünschtenfalls in Abmischung mit Alkalichlorid, in Form einer aufkonzentrierten wäßrigen Salzaufschlämmung mit einem mehrfach-molaren Überschuß an HCl - bezogen auf Alkansulfonsäure -versetzt, die jetzt vorliegende Feststoffphase abtrennt, aus der isolierten Flüssigphase den HCl-Überschuß gewünschtenfalls zusammen mit einem Anteil des Wassers destillativ abzieht und die praktisch chloridfreie Alkansulfonsäure zusammen mit dem Restwasser als Sumpfphase der Destillation gewinnt.

2. Verfahren nach Anspruch 1, dadurch gekennzeichnet, daß man die wäßrige Salzaufschlämmung mit wenigstens etwa 3 Mol HCl, vorzugsweise mit etwa 3,2 bis 4 Mol HCl - jeweils bezogen auf Alkalisalz der Alkansulfonsäure - versetzt, wobei der in der destillativen Trennstufe zurückgewonnene HCl-Anteil bevorzugt im Kreislauf in das Aufarbeitungsverfahren zurückgeführt wird.

3. Verfahren nach Ansprüchen 1 und 2, dadurch gekennzeichnet, daß die Abtrennung des Feststoffanteiles nach der Umsetzung mit HCl bei höchstens mäßig erhöhten Temperaturen, bevorzugt im Temperaturbereich von etwa 40 bis 60 °C durchgeführt wird.

4. Verfahren nach Ansprüchen 1 bis 3, dadurch gekennzeichnet, daß die destillative HCl-Abtrennung mehrstufig, insbesondere zweistufig derart erfolgt, daß zunächst HCl und dann ein HCl/Wasser-Gemisch abgetrennt werden, wobei die Destillation bevorzugt wenigstens anteilsweise im Vakuum bei Temperaturen unterhalb 100 °C durchgeführt wird.

5. Verfahren nach Ansprüchen 1 bis 4, dadurch gekennzeichnet, daß in der zweiten Stufe der destillativen Abtrennung ein HCl/$H_2O$-Azeotrop zurückgewonnen und dabei bevorzugt im schwachen Vakuum im Temperaturbereich von etwa 80 bis 90 °C gearbeitet wird.

6. Verfahren nach Ansprüchen 1 bis 5, dadurch gekennzeichnet, daß in dem wäßrigen, als breiartige Salzaufschlämmung eingestellten Einsatzgemisch solche Wassergehalte vorgegeben werden, daß nach der destillativen Abtrennung des HCl/Wasser-Azeotrops eine freie Alkansulfonsäure mit einem Restwassergehalt von wenigstens etwa 1 bis 30 Gew.-%, bevorzugt von etwa 10 bis 20 Gew.-% als Sumpfphase anfällt.

7. Anwendung des Verfahrens nach Ansprüchen 1 bis 6 zur Gewinnung von $C_{1-4}$-Alkansulfonsäuren, insbesondere der Methansulfonsäure aus ihren Alkalisalzen.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,A D | DE-A-3 812 846 (HENKEL) <br><br> --- | | C 07 C 309/04 <br> C 07 C 303/02 |
| A | US-A-3 480 636 (B. LOEV) <br><br> ----- | | |

RECHERCHIERTE SACHGEBIETE (Int. Cl.5)

C 07 C 303/00
C 07 C 309/00

Der vorliegende Recherchenbericht wurde für alle Patentansprüche erstellt

| Recherchenort | Abschlußdatum der Recherche | Prüfer |
|---|---|---|
| DEN HAAG | 20-09-1990 | VAN GEYT J.J.A. |